(19) Europäisches Patentamt
European Patent Office
Office européen des brevets

(11) Veröffentlichungsnummer: **0 302 018 B1**

(12) **EUROPÄISCHE PATENTSCHRIFT**

(45) Veröffentlichungstag der Patentschrift: **03.11.93**

(51) Int. Cl.[5]: **C07D 487/04**, C08K 5/34, //C09B57/00,(C07D487/04, 209:00,209:00)

(21) Anmeldenummer: **88810505.3**

(22) Anmeldetag: **22.07.88**

Die Akte enthält technische Angaben, die nach dem Eingang der Anmeldung eingereicht wurden und die nicht in dieser Patentschrift enthalten sind.

# (54) Verfahren zur Herstellung von Pyrrolo-(3,4-c)pyrrolen.

(30) Priorität: **31.07.87 CH 2935/87**

(43) Veröffentlichungstag der Anmeldung:
**01.02.89 Patentblatt 89/05**

(45) Bekanntmachung des Hinweises auf die Patenterteilung:
**03.11.93 Patentblatt 93/44**

(84) Benannte Vertragsstaaten:
**CH DE ES FR GB IT LI**

(56) Entgegenhaltungen:
**EP-A- 0 061 426**
**EP-A- 0 094 911**
**EP-A- 0 098 808**
**EP-A- 0 181 290**
**EP-A- 0 184 982**

(73) Patentinhaber: **CIBA-GEIGY AG**
**Klybeckstrasse 141**
**CH-4002 Basel(CH)**

(72) Erfinder: **Surber, Werner, Dr.**
**Vorderbergstrasse 76**
**CH-4104 Oberwil(CH)**
Erfinder: **Iqbal, Abul, Dr.**
**La Dey 202**
**CH-1711 Arconciel(CH)**
Erfinder: **Stern, Christian, Dr.**
**Liebrütistrasse20**
**CH-4303 Kaiseraugst(CH)**

**EP 0 302 018 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein verbessertes Verfahren zur Herstellung von 1,4-Diketopyrrolo-[3,4-c]-pyrrolen, die wertvolle Pigmente darstellen.

Im EP-Patent Nr. 94911 wird ein Verfahren zur Herstellung von 1,4-Diketopyrrolo-[3,4-c]-pyrrolen beschrieben, wonach man 1 Mol eines Bernsteinsäurediesters mit 2 Mol eines einheitlichen Nitrils oder eines Gemisches aus zwei verschiedenen Nitrilen in einem organischen Lösungsmittel in Gegenwart einer starken Base bei erhöhter Temperatur reagieren lässt und aus dem entstandenen Reaktionsprodukt das Pyrrolopyrrol durch Hydrolyse freisetzt. Bei der Hydrolyse- bzw. Protolysestufe werden die in der ersten Verfahrensstufe entstandenen Reaktionsprodukte (Alkali- bzw. Erdalkalisalze der Diketopyrrolopyrrole) auf einmal mit dem Protolysemittel versetzt, wobei hierfür Wasser, ein Alkohol oder eine Säure verwendet werden. Die nach diesem Verfahren erhaltenen Produkte lassen sich als Pigmente im allgemeinen direkt in der Form einsetzen, wie sie nach ihrer Synthese anfallen, obwohl ihre chemische Reinheit nicht immer optimal ist und ihre Pigmenteigenschaften die Anforderungen der Praxis oft nicht voll befriedigen. Eine nachträgliche chemische oder thermische Nachbehandlung der nach diesem Verfahren erhaltenen Produkte ist daher oft erforderlich. Zur Ueberführung in eine deckende Pigmentform beispielsweise müssen diese Produkte meistens in einem organischen Lösungsmittel noch thermisch nachbehandelt werden, was eine zusätzliche getrennte Verfahrensstufe erfordert und zudem noch öfters zur Bildung von unerwünschten Nebenprodukten führt.

Es wurde nun gefunden, dass Pyrrolopyrrolpigmente mit hoher Reinheit und hervorragenden Pigmenteigenschaften direkt erhalten werden können, wenn die Protolyse der nach obigem EP-Patent erhaltenen Reaktionsprodukte sequentiell durchgeführt wird. Dabei lässt sich die Bildung unerwünschter Neben- und Abbauprodukte bei gleichzeitiger Verbesserung der koloristischen Eigenschaften, wie Sättigung, Farbtonreinheit und Glanz sowie der Gebrauchseigenschaften der Pigmente, wie Rheologie, Glanz, Licht- und Wetterbeständigkeit, stark unterdrücken.

Die Erfindung betrifft demnach ein Verfahren zur Herstellung von 1,4-Diketopyrrolo-[3,4-c]-pyrrolen der Formel I

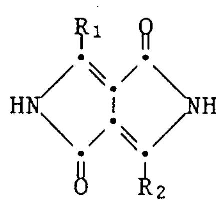

(I),

worin $R_1$ und $R_2$ unabhängig voneinander Phenyl-, Diphenyl- oder Naphthylreste oder aromatische O-, S- oder N-heterocyclische Reste bedeuten, durch Umsetzung von 1 Mol eines Bernsteinsäure-dicyclohexylesters, -dialkylesters, -monoalkylmonophenylesters oder -diphenylesters, wobei im Bernsteinsäureesterrest Alkyl $C_1$-$C_{18}$-Alkyl und Phenyl unsubstituiertes oder durch ein oder zwei Halogenatome, eine oder zwei $C_1$-$C_6$-Alkyl- oder $C_1$-$C_6$-Alkoxygruppen substituiertes Phenyl bedeuten, mit 2 Mol eines Nitrils der Formeln II oder III oder mit 1 Mol eines Nitrils der Formel II und 1 Mol eines Nitrils der Formel III

$R_1$-CN (II) , $R_2$-CN (III),

worin $R_1$ und $R_2$ die oben angegebene Bedeutung haben, in einem inerten organischen Lösungsmittel in Gegenwart eines Alkalimetalls oder eines Alkali-alkoholates als starke Base bei erhöhter Temperatur zu einem Alkalimetallsalz der Formel IV

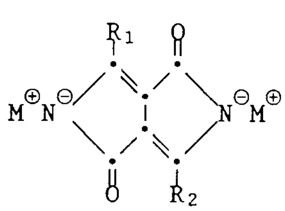

(IV),

worin M ein Alkalimetall darstellt, und anschliessende Freisetzung einer Verbindung der Formel I durch Protolyse einer Verbindung obiger Formel IV, dadurch gekennzeichnet, dass man 1 Gew.-Teil einer Verbindung obiger Formel IV in mindestens zwei Portionen mit 5-20 Gew. -Teilen Wasser, einer anorganischen und/oder organischen Säure, einem Gemisch aus Wasser und einem organischen Lösungsmittel oder einem Gemisch aus einer anorganischen oder organischen Säure und Wasser oder/und mindestens einem organischen Lösungsmittel, als Protolysemittel sequentiell versetzt, und nach Zugabe der jeweiligen Portionen 1 bis 12 Stunden bei einer Temperatur von 20 bis 150 °C reagieren lässt, wobei die Portionengrösse zwischen 10 und 75 Gew.-% der Gesamtmenge an Verbindung der Formel IV variiert.

Bedeuten $R_1$ und $R_2$ Phenyl-, Diphenyl- oder Naphthylreste, so können sie die üblichen nichtwasserlöslichmachenden Substituenten aufweisen, wie z.B.:

1) Halogenatome, beispielsweise Chlor, Brom oder Fluor;

2) Verzweigte oder unverzweigte Alkylgruppen mit vorzugsweise 1 bis 8, insbesondere 1 bis 4 C-Atomen. Beispiele von Alkylgruppen sind Methyl, Aethyl, n-Propyl, Isopropyl, n-Butyl, sec.-Butyl, tert.-Butyl, n-Pentyl, tert.-Amyl, n-Hexyl, 1,1,3,3-Tetramethylbutyl, n-Heptyl und n-Octyl;

3) Die Gruppen -CN, $-CF_3$, $-NO_2$, -COOH, $-CONH_2$ oder $-SO_3H$;

4) Alkoxygruppen enthaltend 1 bis 3 C-Atome, Phenoxy oder durch ein oder Zwei Halogenatome, eine oder zwei Alkyl- oder Alkoxygruppen mit 1 bis 3 C-Atomen substituiertes Phenoxy. Beispiele hierfür sind Methoxy, Aethoxy, n-Propoxy, Phenoxy, o-, m- oder p-Chlorphenoxy, o-, m- oder p-Methylphenoxy, p-Aethoxyphenoxy oder p-Bromphenoxy;

5) Die Gruppe Alkylmercapto mit 1 bis 3 C-Atomen und Phenylmercapto, das durch ein oder zwei Halogenatome, eine oder zwei Alkyl- oder Alkoxygruppen mit 1 bis 3 C-Atomen substituiert sein kann. Beispiele für Alkylmercapto sind Methyl-, Aethyl- und n-Propylmercapto; Beispiele für Phenylmercapto sind Phenyl-, p-Chlorphenyl- und p-Methylphenylmercapto;

6) Die Gruppe Dimethyl- oder Diäthylamino;

7) Die Gruppe Alkoxycarbonyl enthaltend 2 bis 5 C-Atome. Beispiele hierfür sind Methoxy-, Aethoxy-, n-Propoxy- und n-Butoxy-carbonyl;

8) Die Gruppe $C_2$-$C_5$-Alkanoylamino, wie z.B. Acetylamino, Propionylamino oder Butyrylamino, ferner Benzoylamino, das durch ein oder zwei Halogenatome, eine oder zwei Alkyl- oder Alkoxygruppen mit 1 bis 3 C-Atomen substituiert sein kann, wie z.B. Benzoylamino, p-Chlorbenzoylamino und p-Methylbenzoylamino.

Bedeuten $R_1$ und $R_2$ Reste aromatischer O-, S- oder N-Heterocyclen, so können sie unsubstituiert oder z.B. durch Halogen, wie Chlor, Brom oder Fluor, Alkyl oder Alkoxy enthaltend 1 bis 3 C-Atome substituiert sein. Beispiele für derartige Heterocyclen sind Chinolyl-, Isochinolyl-, o-, m- oder p-Pyridyl-, Furanyl-, Thiofuranyl-, Benzoxazolyl-, Benzthiazolyl- und Benzimidazolylreste.

Bevorzugte N-heterocyclische Reste sind o-, m- und p-Pyridyl.

Zur erfindungsgemässen Herstellung der Verbindungen der Formel I verwendet man als Ausgangsstoff vorzugsweise ein einheitliches Nitril der Formel II oder III, wobei $R_1$ und $R_2$ Phenyl- oder Naphthyl-Radikale bedeuten.

Bevorzugt verwendet man als Ausgangsstoffe Nitrile der Formel V

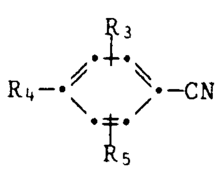

(V),

worin, $R_3$, $R_4$ und $R_5$ unabhängig voneinander Wasserstoff, Fluor, Chlor, Brom, Carbamoyl, Cyano, Trifluormethyl, $C_1$-$C_8$-Alkyl, $C_1$-$C_3$-Alkoxy, $C_1$-$C_3$-Alkylmercapto, $C_2$-$C_5$-Alkoxycarbonyl, $C_2$-$C_5$-Alkanoylamino, N-Dimethyl- oder -Diäthylamino, unsubstituiertes oder durch ein oder zwei Halogenatome, eine oder zwei $C_1$-$C_3$-Alkyl- oder $C_1$-$C_3$-Alkoxygruppen substituiertes Phenoxy, Phenylmercapto oder Benzoylamino bedeuten, wobei mindestens einer der Substituenten $R_3$, $R_4$ und $R_5$ Wasserstoff bedeutet.

Ganz bevorzugt verwendet man als Ausgangsstoffe Nitrile der Formel VI

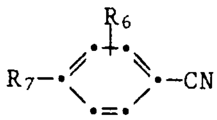

(VI),

worin $R_6$ und $R_7$ unabhängig voneinander Wasserstoff, Chlor, Brom, Fluor, Methyl, Methoxy, Aethoxy, Cyan, unsubstituiertes oder durch Chlor oder Methyl substituiertes Phenoxy oder Phenylmercapto, Methoxy- oder Aethoxycarbonyl, Acetylamino oder unsubstituiertes oder durch Chlor, Methyl oder Methoxy unsubstituiertes Benzoylamino bedeuten.

Ganz besonders verwendet man als Ausgangsprodukte Nitrile obiger Formel VI, worin einer der Substituenten $R_6$ und $R_7$ die oben angegebene Bedeutung hat, und der andere Wasserstoff bedeutet.

Bei den zu verwendenden Bernsteinsäuredialkyl- oder -diphenylestern kann es sich um symmetrische oder asymmetrische Diester handeln. Bevorzugt verwendet man aber symmetrische Bernsteinsäurediester, insbesondere symmetrische Bernsteinsäuredialkylester. Liegt ein Bernsteinsäurediphenyl- oder -monophenylmonoalkylester vor, so kann Phenyl beispielsweise unsubstituiertes oder durch ein oder zwei Halogenatome, wie Chlor, $C_1$-$C_6$-Alkylgruppen, wie Methyl, Aethyl, Isopropyl oder tert.-Butyl, oder $C_1$-$C_6$-Alkoxygruppen, wie Methoxy oder Aethoxy, substituiertes Phenyl bedeuten. Phenyl bedeuten bevorzugt unsubstituiertes Phenyl. Handelt es sich um einen Bernsteinsäuredialkyl- oder -monoalkylmonophenylester, so kann Alkyl unverzweigt oder verzweigt sein, bevorzugt verzweigt, und vorzugsweise 1 bis 12, insbesondere 1 bis 8 und besonders bevorzugt 1 bis 5 C-Atome enthalten. Verzweigtes Alkyl ist bevorzugt sek.- oder tert.-Alkyl, wie z.B. Isopropyl, sek.-Butyl, tert.-Butyl und tert.-Amyl. Ganz bevorzugt verwendet man symmetrische verzweigte Bernsteinsäuredialkylester, worin jeder Alkyl im Bernsteinsäureesterrest 3 bis 5 C-Atome aufweist.

Beispiele für Bernsteinsäurediester sind Bernsteinsäuredimethylester, -diäthylester, -dipropylester, -dibutylester, -dipentylester, -dihexylester, -diheptylester, -dioctylester, -diisopropylester, -di-sec.-butylester, -di-tert.-butylester, -di-tert.-amylester, -di-[1,1-dimethylbutyl]-ester, -di-[1,1,3,3-tetramethylbutyl]-ester, -di-[1,1-dimethylpentyl]-ester, -di-[1-methyl-1-äthyl-butyl]-ester, -di-[1,1-diäthylpropyl]-ester, -diphenylester, -di-[4-methylphenyl]-ester, -di-[2-methylphenyl]-ester, -di-[4-chlorphenyl]-ester, -di-[2,4-dichlorphenyl]-estern und -monoäthyl-monophenylester.

Die oben aufgeführten Bernsteinsäurediester und die Nitrile der Formel II bzw. III sind bekannte Verbindungen und können nach bekannten Verfahren hergestellt werden.

Man führt die Umsetzung des Bernsteinsäurediesters mit dem Nitril der Formeln II oder III bzw. einem Gemisch davon in einem inerten organischen Lösungsmittel durch. Als Lösungsmittel eignen sich beispielsweise primäre, sekundäre oder tertiäre Alkohole mit 1 bis 10 C-Atomen, wie Methanol, Aethanol, n-Propanol, Isopropanol, n-Butanol, sek.-Butanol, tert.-Butanol, n-Pentanol, 2-Methyl-2-butanol, 2-Methyl-2-pentanol, 3-Methyl-3-pentanol, 2-Methyl-2-hexanol, 3-Aethyl-3-pentanol und 2,4,4-Trimethyl-2-pentanol, Glykole, wie Aethylenglykol oder Diäthylenglykol, ferner Aether, wie Tetrahydrofuran oder Dioxan, oder Glykoläther, wie Aethylenglykol-mono- oder -di-methyläther, Aethylenglykol-mono- oder -di-äthyläther, Diäthylenglykol-monomethyläther oder Diäthylenglykol-monoäthyläther, ferner dipolar-aprotische Lösungsmittel, wie Acetonitril, Benzonitril, Dimethylformamid, N,N-Dimethylacetamid, Nitrobenzol und N-Methylpyrrolidon, aliphatische oder aromatische Kohlenwasserstoffe, wie Benzol oder durch Alkyl, Alkoxy oder Halogen substituiertes Benzol, wie Toluol, Xylole, Anisol oder Chlorbenzol, oder aromatische N-Heterocyclen, wie Pyridin, Picolin oder Chinolin. Zudem ist es auch möglich, das umzusetzende Nitril der Formel II bzw. III gleichzeitig als Lösungsmittel im Ueberschuss zu verwenden, falls es im Temperaturbereich flüssig ist, in dem die Umsetzung erfolgt. Die oben genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Im erfindungsgemässen Verfahren verwendet man als Lösungsmittel bevorzugt einen Alkohol, insbesondere einen sekundären oder tertiären Alkohol. Bevorzugte tertiäre Alkohole sind tert.-Butanol und tert.-Amylalkohol. Dabei sind auch Gemische davon, oder Gemische dieser bevorzugten Lösungsmittel mit aromatischen Kohlenwasserstoffen, wie Toluol oder Xylole, oder mit durch Halogen substituierten Benzolen, wie Chlorbenzol oder o-Dichlorbenzol, von grossem Interesse.

Anmeldungsgemäss geeignete starke Basen sind Alkalimetalle, wie Lithium, Natrium und Kalium, und Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -äthylat, -n-propylat, -isopropylat, -n-butylat, -sek.-butylat, -tert.-butylat, -2-methyl-2-butylat, -2-methyl-2-pentylat, -3-methyl-3-pentylat und -3-äthyl-3-pentylat. Man kann aber auch ein Gemisch der oben genannten Alkalialkoholate verwenden. Bevorzugt verwendet man Alkalialkoholate, wobei Alkali insbesondere Natrium oder Kalium bedeutet, und das Alkoholat sich bevorzugt von einem sekundären oder tertiären Alkohol ableitet. Besonders bevorzugte starke Basen sind daher z.B. Natrium- oder Kaliumisopropylat, -sek.-butylat, -tert.-butylat und -tert.-amylat. Dabei können die Alkalialkoholate auch in situ hergestellt werden, indem man den entsprechenden Alkohol mit dem Alkalimetall umsetzt.

Im erfindungsgemässen Verfahren kann man die starke Base beispielsweise in einer Menge von 0,1 bis 10 Mol, bevorzugt 1,9 bis 4,0 Mol, bezogen auf 1 Mol des Bernsteinsäurediesters, einsetzen. Obwohl grundsätzlich stöchiometrische Mengen an Base genügen, beeinflusst in vielen Fällen ein Basenüberschuss die Ausbeute günstig.

Die Umsetzung kann beispielsweise bei einer Temperatur von 60 bis 140 °C, vorzugsweise aber von 80 bis 120 °C, durchgeführt werden.

Zur Umsetzung des Bernsteinsäurediesters mit dem bzw. den Nitrilen der Formeln II, III, V und VI ist es grundsätzlich möglich, bei tieferer Temperatur alle Komponenten vorzulegen und dann das Gemisch in den Bereich der Reaktionstemperatur zu erwärmen, oder in beliebiger Reihenfolge die einzelnen Komponenten im Bereich der Reaktionstemperatur zuzugeben. Eine bevorzugte Ausführungsform, die sich in der Regel besonders günstig auf die Ausbeute auswirkt, besteht darin, dass man das umzusetzende Nitril zusammen mit der starken Base vorlegt und den Bernsteinsäurediester im Bereiche der Reaktionstemperatur zudosiert. Eine weitere Möglichkeit besteht darin, dass man den Bernsteinsäurediester und das umzusetzende Nitril gleichzeitig zur vorgelegten Base zudosiert. Es ist durchaus möglich, das erfindungsgemässe Verfahren nicht nur batchweise, sondern auch kontinuierlich durchzuführen.

Insbesondere bei Bernsteinsäurediestern mit niederen Alkylresten und bei Alkoholaten, die sich aus niederen Alkoholen ableiten, wie z.B. Methanol, Aethanol, n-Propanol, Isopropanol oder tert.-Butanol, kann es sich als vorteilhaft erweisen, den bei der Umsetzung entstehenden niederen Alkohol laufend aus dem Reaktionsmedium zu entfernen, um höhere Ausbeuten zu erzielen.

Verwendet man als Lösungsmittel einen Alkohol und als Base ein Alkoholat, so kann es vorteilhaft sein, einen Alkohol und ein Alkoholat mit gleichen Alkylteilen zu wählen. Ebenso vorteilhaft kann es sein, wenn zudem noch der Bernsteinsäurediester ebensolche Alkylgruppen enthält.

Zur Protolyse der erhaltenen Kondensationsprodukte der Formel IV kann man das erfindungsgemäss in Frage kommende Protolysemittel in beliebigen Mischungsverhältnissen zwischen 5 und 20 Gew.-Teilen auf 1 Teil einer Verbindung der Formel IV verwenden.

Organische Säuren als Protolysemittel sind z.B. aliphatische oder aromatische Carbon- oder Sulfonsäuren, wie z.B. Ameisensäure, Essigsäure, Propionsäure, Buttersäure, Hexansäure, Oxalsäure, Benzoesäure, Phenylessigsäure, Benzolsulfonsäure oder p-Toluolsulfonsäure.

Als anorganische Säure kommen beispielsweise Salzsäure, Schwefelsäure oder Phosphorsäure in Frage.

Gemische einer anorganischen und organischen Säure sind z.B. Gemische aus den oben erwähnten Säuren.

Erfindungsgemäss geeignete organische Lösungsmittel als Protolysemittel sind im Prinzip die gleichen Lösungsmittel, welche bereits oben bei der ersten Verfahrensstufe (Kondensationsstufe) aufgeführt sind. Bevorzugt sind Alkohole mit 1 bis 5 C-Atomen, wie Methanol, Aethanol, Propanol, Isopropanol, tert.-Butylalkohol und tert.-Amylalkohol.

Erfindungsgemäss bevorzugte Protolysemittel sind Wasser, Gemische aus Wasser und einem aliphatischen Alkohol, insbesondere Methanol, Aethanol oder tert.-Amylalkohol, ferner Wasser im Gemisch mit einer Säure, insbesondere Essigsäure, HCl oder $H_2SO_4$, oder ein Gemisch aus Wasser, einer Säure und einem organischen Lösungsmittel, wie z.B. Wasser, Salzsäure oder Essigsäure, und Methanol oder Aethanol oder tert.-Amylalkohol oder Toluol oder Xylol. Ganz besonders bevorzugt verwendet man Wasser allein oder Wasser-Methanol-Gemische, in beliebigen Verhältnissen.

Zur Protolyse des Reaktionsproduktes der Formel IV wird das Protolysemittel vorgelegt und dann das Produkt der Formel IV in mindestens zwei Portionen zugegeben. Die Portionengrösse variiert zwischen 10 und 75 Gewichtsprozent der Gesamtmenge am Produkt der Formel IV, wobei nach jeder Portionszugabe während einer bis mehrerer Stunden nachgerührt wird, gegebenenfalls unter Druck.

Die Reaktionstemperatur bei der Protolysestufe liegt bevorzugt zwischen 50 und 100 °C. Die Reaktionszeit beträgt, je nach Ansatzgrösse, 1 bis 12 Stunden, bevorzugt 1 bis 8 Stunden.

Wird die Protolysestufe ohne Wasser durchgeführt, so beträgt die Säuremenge zweckmässig 1 bis 4 Aequivalente, bevorzugt 1 bis 2 Aequivalente, bezogen auf die Menge des Alkalisalzes der Formel IV.

Erfindungsgemäss bevorzugt wird die Protolysestufe in 2 Portionen durchgeführt, wobei die erste Portion der Verbindung der Formel IV 40 bis 50 Gew.-% der Gesamtmenge beträgt.

Nach der Protolyse fallen die Verbindungen der Formel I aus und können durch an und für sich bekannte Trennverfahren, wie Abfiltrieren, isoliert werden.

Je nach Art ihrer Substituenten und der zu färbenden Polymeren können die Verbindungen der Formel I auch als polymerlösliche Farbstoffe verwendet werden. In der Regel jedoch verwendet man sie als Pigmente für hochmolekulare organische Materialien. Dabei lassen sich die Pigmente meistens direkt in der Pigmentform einsetzen, wie sie nach dem erfindungsgemässen Verfahren anfallen. Ihre Kristallmorphologie kann je nach Verwendungszweck, und sofern notwendig, durch eine nachträgliche Behandlung noch optimiert werden. So kann man das Pigment beispielsweise nach der Protolysestufe zuerst isolieren und nachträglich in Wasser oder in einem organischen Lösungsmittel erwärmen, gegebenfalls unter Druck, um beispielsweise eine deckende bzw. deckendere Form zu erlangen. Vorzugsweise verwendet man organische Lösungsmittel, die über 80°C sieden. Als besonders geeignet erweisen sich durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Aether, wie Aethylenglykolmonomethyl-oder -monoäthyläther, Amide, wie Dimethylformamid oder N-Methylpyrrolidon, sowie Dimethylsulfoxid oder Sulfolan. Man kann die Nachbehandlung auch in Wasser in Gegenwart von organischen Lösungsmitteln und/oder mit Zusatz von oberflächenaktiven Substanzen durchführen.

Je nach Verwendungszweck kann es vorteilhaft sein, Gemische von Verbindungen der Formel I herzustellen. Dies lässt sich beispielsweise dadurch erreichen, dass man unabhängig voneinander hergestellte, verschiedene Reaktionslösungen vor der Protolyse mischt, sie zusammen protolysiert und dann das erhaltene Produkt isoliert.

Hochmolekulare organische Materialien, die mit den Verbindungen der Formel I gefärbt, bzw. pigmentiert werden können, sind z.B. Celluloseäther und -ester, wie Aethylcellulose, Nitrocellulose, Celluloseacetat und Cellulosebutyrat, natürliche Harze und Kunstharze, wie Polymerisationsharze oder Kondensationsharze, z.B. Aminoplaste, insbesondere Harnstoff-und Melamin-Formaldehydharze, Alkydharze, Phenoplaste, Polycarbonate, Polyolefine, wie Polyäthylen und Polypropylen, Polystyrol, Polyvinylchlorid, Polyacrylnitril, Polyacrylsäureester, Polyamide, Polyurethane, Polyester, Gummi, Casein, Silikon und Silikonharze, einzeln oder in Mischungen.

Dabei spielt es keine Rolle, ob die erwähnten hochmolekularen organischen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die Verbindungen der Formel I als Toner oder in Form von Präparaten einzusetzen. Bezogen auf das zu pigmentierende hochmolekulare organische Material kann man die Verbindungen der Formel I in einer Menge von 0,01 bis 30 Gew.-%, vorzugsweise 0,1 bis 10 Gew.-%, einsetzen.

Die erhaltenen Ausfärbungen, beispielsweise in Kunststoffen, Fasern, Lacken oder Drucken, zeichnen sich durch eine gute Dispergierbarkeit, hohe Farbstärke, gute Ueberlackier-, Migrations-, Hitze-, Licht- und Wetterbeständigkeit, sowie durch einen guten Glanz, aus.

In den nachfolgenden Beispielen bedeuten die Teile Gewichtsteile.

Beispiel 1: In eine aus 90,1 g Natrium und 1560 ml tert.-Amylalkohol hergestellte Alkoholat-Lösung werden unter Stickstoffatmosphäre 357,9 g 4-Chlorbenzonitril hinzugegeben, und das erhaltene Gemisch wird auf 85°C erwärmt. Anschliessend werden innerhalb 80 Minuten bei 85-95°C 262,6 g Bernsteinsäure-diisopropylester zugetropft, und die erhaltene Suspension wird bei dieser Temperatur weitere zwei Stunden gerührt. Die Hälfte der entstandenen Pyrrolopyrrol-Natriumsalz-Suspension (226,5 g) wird auf 700 ml Wasser ausgetragen und während 8 Stunden am Rückfluss gerührt. Nun werden die restlichen 50 Prozente (= 226,5 g) obiger Natriumsalz-Suspension bei 85°C hinzugegeben, und die erhaltene Suspension wird während einer Stunde am Rückfluss gerührt.

Danach wird zur vollständigen Entfernung des tert.-Amylalkohols das Reaktionsgemisch einer Wasserdampfdestillation unterworfen. Die zurückbleibende wässerige Suspension wird heiss filtriert, und der Filterkuchen wird mit 6 Litern warmen Wasser neutral gewaschen und im Trockenschrank bei 120°C unter Vakuum getrocknet. Man erhält auf diese Weise ein rotes Pigment der folgenden Formel

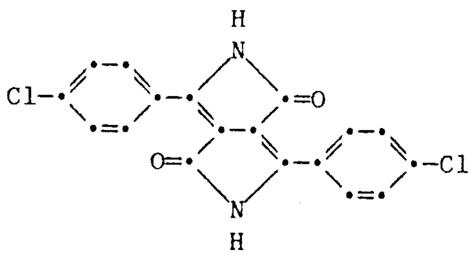

,

das ausgezeichnete koloristische und Pigment-Eigenschaften aufweist.

Beispiele 2 bis 17: Arbeitet man analog dem Beispiel 1 mit Bernsteinsäure-di-tert.-butylester anstatt -diisopropylester unter den in der folgenden Tabelle 1 aufgeführten Bedingungen für die sequentielle Protolyse, so erhält man jeweils Produkte mit ausgezeichneten Pigment-eigenschaften. Dabei lässt sich die Bildung unerwünschter Neben- und Abbauprodukte stark unterdrücken.

Tabelle 1

| Beispiel Nr. | Sequentielle Protolyse der Natriumsalz-Suspension | | | | Protolysemittel |
|---|---|---|---|---|---|
| | erste Portion | | zweite Portion | | |
| | Menge in Gew.-% der Na-Salz-Susp. | Nachrühren Dauer (Std.) | Menge in % der Na-Salz-Susp. | Nachrühren Dauer (Std.) | |
| 2 | 50 | 7 | 50 | 1 | Wasser |
| 3 | 50 | 4 | 50 | 1 | Wasser |
| 4 | 50 | 4 | 50 | 8 | Wasser |
| 5 | 50 | 8 | 50 | 1 | Wasser |
| 6 | in 8 gewichtsgleichen Portionen über 8 Std. hinzugegeben | | | | Wasser |
| 7 | 50 | 1 | 50 | 5 | Wasser |
| 8 | 50 | 1 | 50 | 8 | Wasser |
| 9 | 25 | 8 | 75 | 1 | Wasser |
| 10 | 25 | 4 | 75 | 5 | Wasser |
| 11 | 75 | 4 | 25 | 4 | Wasser |
| 12 | 50 | 8 | 50 | 1 (1) | Wasser |
| | | | | | Methanol: Wasser (2) |
| 13 | 50 | 4 | 50 | 8 | 1 : 4 |
| 14 | 50 | 2 | 50 | 2 | 1 : 1 |
| 15 | 50 | 4 | 50 | 8 | 4 : 1 |

Tabelle 1 (Fortsetzung)

| Beispiel Nr. | Sequentielle Protolyse der Natriumsalz-Suspension: erste Portion: Menge in Gew.-% der Na-Salz-Susp. | erste Portion: Nachrühren Dauer (Std.) | zweite Portion: Menge in % der Na-Salz-Susp. | zweite Portion: Nachrühren Dauer (Std.) | Protolysemittel |
|---|---|---|---|---|---|
| 16 | 50 | 6 | 50 | 3 | Methanol/Eisessig(3) (% d.Stöch.) 50 |
| 17 | 50 | 4 | 50 | 4 | tert.-Amylalkohol/Eisessig (% d.Stöch.) 50 |

(1) Die Konditionierungstemperatur nach der Zugabe der 2. Hälfte der Natriumsalzsuspension beträgt 50°C anstatt 85-90°C (Rückflusstemperatur);

(2) Volumenverhältnis;

(3) % der Stöch. = % der zur vollständigen Neutralisation stöchiometrisch erforderlichen Menge Eisessig.

Beispiele 18-22: Arbeitet man analog dem Beispiel 1 mit Bernsteinsäure-di-tert.-butylester anstatt Bernsteinsäure-di-isopropylester unter den in der folgenden Tabelle 2 aufgeführten Bedingungen für die Protolysestufe, so erhält man jeweils Produkte mit ausgezeichneten Pigmenteigenschaften.

Tabelle 2

| Beispiel Nr. | Sequentielle Protolyse der Natriumsalz-Suspension* | | | | Protolysemittel | |
|---|---|---|---|---|---|---|
| | erste Portion | | zweite Portion | | | |
| | Menge in Gew.-% der Na-Salz-Susp. | Nachrühren Dauer (Std.) | Menge in Gew.-% der Na-Salz-Susp. | Nachrühren Dauer (Std.) | Bestandteile | Volumen-verhältnis |
| 18 | 50 | 2 | 50 | 2 | Wasser/Methanol und Eisessig (50 % d. Stöchiometrie**) | 1:1 |
| 19 | 50 | 6 | 50 | 3 | Wasser + Eisessig (100 % d. Stöchiometrie**) | |
| 20 | 50 | 4 | 50 | 8 | Eisessig/Wasser | 4:1 |
| 21 | 50 | 4 | 50 | 8 | Dioxan/Wasser | 4:1 |
| 22 | 50 | 4 | 50 | 8 | Aethanol/Wasser | 4:1 |

* Konditionierungstemperatur nach jeweiliger Protolyse der Na-Salz-Suspension = Rückflusstemperatur des jeweils vorliegenden Reaktionsgemisches.

** % der Stöchiometrie = % der zur vollständigen Neutralisation stöchiometrisch erforderlichen Menge Eisessig.

Beispiele 23-28: Arbeitet man analog dem Beispiel 1 mit Bernsteinsäure-di-tert.-butylester anstatt Bernsteinsäure-di-isopropylester mit einem in Tabelle 3 aufgeführten Nitril der Formel ArCN anstatt p-Chlorbenzonitril, so erhält man unter den in Tabelle 3 erwähnten Protolysebedingungen jeweils ein Produkt der Formel

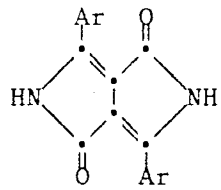

mit ausgezeichneten Pigmenteigenschaften.

Tabelle 3

| Bei-spiel Nr. | Nitril (Ar-CN) | Sequentielle Protolyse der Natriumsalz-Suspension* | | | | Protolysemittel | |
|---|---|---|---|---|---|---|---|
| | | erste Portion | | zweite Portion | | | |
| | | Menge in Gew.-% der Na-Salz-Susp. | Nachrühren Dauer (Std.) | Menge in Gew.-% der Na-Salz-Susp. | Nachrühren Dauer (Std.) | Bestandteile | Volumen-verhält-nis |
| 23 | 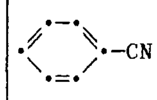 | 50 | 2 | 50 | 2 | (gemäss Beispiel 1) | |
| 24 | 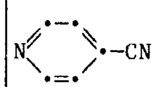 | 50 | 2 | 50 | 2 | Methanol/Wasser und Eisessig (100 % d. Stöchiometrie**) | 4:1 |
| 25 | 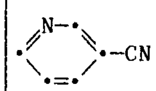 | 50 | 2 | 50 | 2 | Methanol/Wasser und Eisessig (100 % d. Stöchiometrie**) | 4:1 |
| 26 | 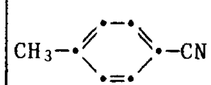 | 50 | 2 | 50 | 2 | Methanol/Wasser | 4:1 |
| 27 | 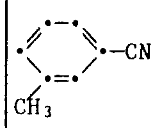 | 50 | 2 | 50 | 2 | Methanol/Wasser | 4:1 |

Tabelle 3 (Fortsetzung)

| Beispiel Nr. | Nitril (Ar-CN) | Sequentielle Protolyse der Natriumsalz-Suspension* erste Portion: Menge in Gew.-% der Na-Salz-Susp. | erste Portion: Nachrühren Dauer (Std.) | zweite Portion: Menge in Gew.-% der Na-Salz-Susp. | zweite Portion: Nachrühren Dauer (Std.) | Protolysemittel: Bestandteile | Protolysemittel: Volumenverhältnis |
|---|---|---|---|---|---|---|---|
| 28 | 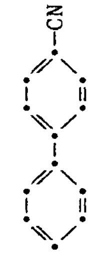 | 50 | 4 | 50 | 4 | Methanol/Wasser | 4:1 |

* Konditionierungstemperatur der jeweiligen Protolyse-Suspension: Beispiel 23 = 40°C; Beispiele 24-28 = Rückflusstemperatur des jeweils vorliegenden Reaktionsgemisches.

** % der Stöchiometrie = % der zur vollständigen Neutralisation stöchiometrisch erforderlichen Menge Eisessig.

## Anwendungsbeispiele

Beispiel 29 (Weich-Polyvinylchlorid): 0,6 g des Pigments gemäss obigem Beispiel 1 werden mit 67 g Polyvinylchlorid, 33 g Dioctylphthalat, 2 g Dibutylzinndilaurat und 2 g Titandioxyd zusammengemischt, und

das erhaltene Gemisch wird auf dem Walzenstuhl während 15 Minuten bei 160 °C zu einer dünnen Folie verarbeitet. Die so erzeugte rote PVC-Folie ist farbstark, migrations- und lichtbeständig.

Beispiel 30 (Polyäthylen): 0,2 Teile des Pigments gemäss obigem Beispiel 1, 1 Teil Titandioxyd (Rutil) und 100 Teile LD-Polyäthylengranulat werden in einer Trommel gemischt, und das Gemisch wird anschliessend auf dem Mischwalzwerk bei 130 °C verarbeitet. Die Masse wird heiss zu Platten verpresst oder in der Strangpresse verformt. Die Platten zeigen eine schöne rote Färbung von guter Lichtbeständigkeit.

Beispiel 31 (Alkyd-Melamin-Einbrennlack): 60 Teile einer 60 %-igen Lösung eines nicht-trochnenden Alkydharzes in Xylol (Handelsname ®Beckosol 27-320 der Firma Reichhold-Albert-Chemie), 36 Teile einer 50 %-igen Lösung eines Melamin-Formaldehyd-Harzes in einem Butanol-Xylol-Gemisch (Handelsname ®Super-Beckamin 13-501 der Firma Reichhold-Albert-Chemie), 2 Teile Xylol und 2 Teile Glykol-monomethyläther werden vermischt und 100 Teile dieses Gemisches werden mit Hilfe eines Rührers zu einer homogenen Lacklösung verrührt.

95 Teile des so erhaltenen Klarlackes und 5 Teile des Pigmentes gemäss obigem Beispiel 1 werden in einer Kugelmühle während 72 Stunden dispergiert. Der eingefärbte Lack wird dann nach üblicher Spritzmethode auf Blech appliziert und 30 Minuten bei 120 °C eingebrannt. Man erhält eine Lackierung von guter Lichtbeständigkeit.

**Patentansprüche**

**1.** Verfahren zur Herstellung von 1,4-Diketopyrrolo-[3,4-c]-pyrrolen der Formel I

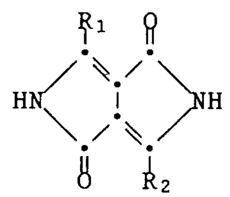

(I),

worin $R_1$ und $R_2$ unabhängig voneinander Phenyl-, Diphenyl- oder Naphthylreste oder aromatische O-, S- oder N-heterocyclische Reste bedeuten, durch Umsetzung von 1 Mol eines Bernsteinsäuredicyclohexylesters, -dialkylesters, -monoalkylmonophenylesters oder -diphenylesters, wobei im Bernsteinsäureesterrest Alkyl $C_1$-$C_{18}$-Alkyl und Phenyl unsubstituiertes oder durch ein oder zwei Halogenatome, eine oder zwei $C_1$-$C_6$-Alkyl- oder $C_1$-$C_6$-Alkoxygruppen substituiertes Phenyl bedeuten, mit 2 Mol eines Nitrils der Formel II oder III oder mit 1 Mol eines Nitrils der Formel II und 1 Mol eines Nitril der Formel III

$R_1$-CN (II) , $R_2$-CN (III),

worin $R_1$ und $R_2$ die oben angegebene Bedeutung haben, in einem inerten organischen Lösungsmittel in Gegenwart eines Alkalimetalls oder eines Alkali-alkoholates als starke Base bei erhöhter Temperatur zu einem Alkalimetallsalz der Formel IV

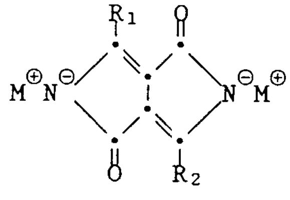

(IV),

worin M ein Alkalimetall darstellt, und anschliessende Freisetzung einer Verbindung der Formel I durch Protolyse einer Verbindung obiger Formel IV, dadurch gekennzeichnet, dass man 1 Gew.-Teil einer Verbindung obiger Formel IV in mindestens zwei Portionen mit 5-20 Gew.-Teilen Wasser, einer anorganischen und/oder organischen Säure, einem Gemisch aus Wasser und einem organischen Lösungsmittel, oder einem Gemisch aus einer anorganischen oder organischen Säure und Wasser oder/und mindestens einem organischen Lösungsmittel, als Protolysemittel sequentiell versetzt, und nach Zugabe der jeweiligen Portionen 1 bis 12 Stunden bei einer Temperatur von 20 bis 150 °C

reagieren lässt, wobei die Portionengrösse zwischen 10 und 75 Gew.% der Gesamtmenge an Verbindung der Formel IV variiert.

**2.** Verfahren nach Anspruch 1, dadurch gekennzeichnet, dass man als Ausgangsstoff ein einheitliches Nitril der Formel II oder III verwendet, wobei $R_1$ und $R_2$ Phenyl- oder Naphthylradikale bedeuten.

**3.** Verfahren nach Anspruch 1, dadurch gekennzeichnet, dass man als Ausgangsstoffe Nitrile der Formel II oder III verwendet, worin $R_1$ und $R_2$ als aromatische N-heterocyclische Reste o-, m- oder p-Pyridyl bedeuten.

**4.** Verfahren nach Anspruch 1, dadurch gekennzeichnet, dass man als Ausgangsstoffe Nitrile der Formel V

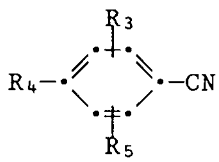

(V)

verwendet,
worin $R_3$, $R_4$ und $R_5$ unabhängig voneinander Wasserstoff, Fluor, Chlor, Brom, Carbamoyl, Cyano, Trifluormethyl, $C_1$-$C_8$-Alkyl, $C_1$-$C_3$-Alkoxy, $C_1$-$C_3$-Alkylmercapto, $C_2$-$C_5$-Alkoxycarbonyl, $C_2$-$C_5$-Alkanoylamino, N-Dimethyl- oder -Diäthylamino, unsubstituiertes oder durch ein oder zwei Halogenatome, eine oder zwei $C_1$-$C_3$-Alkyl- oder $C_1$-$C_3$-Alkoxygruppen substituiertes Phenoxy, Phenylmercapto oder Benzoylamino bedeuten, wobei mindestens einer der Substituenten $R_3$, $R_4$ und $R_5$ Wasserstoff bedeutet.

**5.** Verfahren nach Anspruch 1, dadurch gekennzeichnet, dass man als Ausgangsstoffe Nitrile der Formel VI

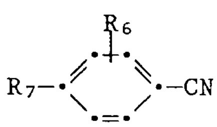

(VI)

verwendet, worin $R_6$ und $R_7$ unabhängig voneinander Wasserstoff, Chlor, Brom, Fluor, Methyl, Methoxy, Aethoxy, Cyan, unsubstituiertes oder durch Chlor oder Methyl suhstituiertes Phenoxy oder Phenylmercapto, Methoxy- oder Aethoxycarbonyl, Acetylamino oder unsubstituiertes oder durch Chlor, Methyl oder Methoxy substituiertes Benzoylamino bedeuten.

**6.** Verfahren nach Anspruch 5, dadurch gekennzeichnet, dass einer der Substituenten $R_6$ und $R_7$ die im Anspruch 5 angegebene Bedeutung hat, und der andere Wasserstoff ist.

**7.** Verfahren nach Anspruch 1, dadurch gekennzeichnet, dass man als Bernsteinsäuredialkylester einen symmetrischen verzweigten Bernsteinsäuredialkylester verwendet, worin jeder Alkylrest im Bernsteinsäureesterrest 3 bis 5 C-Atome aufweist.

**8.** Verfahren nach Anspruch 1, dadurch gekennzeichnet, dass man als Lösungsmittel für die erste Reaktionsstufe einen sekundären oder tertiären Alkohol verwendet.

**9.** Verfahren nach Anspruch 1, dadurch gekennzeichnet, dass man als starke Base ein Alkalialkoholat verwendet.

**10.** Verfahren nach Anspruch 1, dadurch gekennzeichnet, dass die Umsetzung bei der ersten Reaktionsstufe bei einer Temperatur von 60 bis 140 °C durchgeführt wird.

**11.** Verfahren nach Anspruch 1, dadurch gekennzeichnet, dass man als organisches Lösungsmittel für die Protolyse Alkohole mit 1 bis 5 C-Atomen verwendet.

**12.** Verfahren nach Anspruch 1, dadurch gekennzeichnet, dass man als Protolysemittel Wasser, Gemische aus Wasser und einem aliphatischen Alkohol, Wasser im Gemisch mit einer Säure, oder ein Gemisch aus Wasser, einer Säure und einem organischen Lösungsmittel, verwendet.

**13.** Verfahren nach Anspruch 12, dadurch gekennzeichnet, dass man Wasser allein oder Wasser-Methanol-Cemische verwendet.

**14.** Verfahren nach Anspruch 1, dadurch gekennzeichnet, dass die Protolysestufe in zwei Portionen durchgeführt wird, wobei die erste Portion der Verbindung der Formel IV 40 bis 50 Gew.-% der Gesamtmenge beträgt.

**15.** Verfahren nach Anspruch 1, dadurch gekennzeichnet, dass die Temperatur bei der Protolysestufe zwischen 50 und 100 ° C liegt.

**Claims**

**1.** Process for the preparation of a 1,4-diketopyrrolo[3,4-c]pyrrole of the formula I

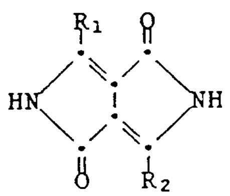

(I)

in which $R_1$ and $R_2$ independently of one another are phenyl, biphenyl or naphthyl radicals or aromatic O-heterocyclic, S-heterocyclic or N-heterocyclic radicals, by reacting 1 mole of a dicyclohexyl succinate, dialkyl succinate, monoalkylmonophenyl or diphenyl succinate, alkyl in the succinic acid ester radical being $C_1$-$C_{18}$alkyl and phenyl being phenyl which is unsubstituted or substituted by one or two halogen atoms or one or two $C_1$-$C_6$alkyl or $C_1$-$C_6$alkoxy groups, with 2 moles of a nitrile of the formula II or III or with 1 mole of a nitrile of the formula II and 1 mole of a nitrile of the formula III

$R_1$-CN (II), $R_2$-CN (III)

in which $R_1$ and $R_2$ are as defined above, in an inert organic solvent, in the presence of an alkali metal or an alkali metal alcoholate, as a strong base, and at an elevated temperature to give an alkali metal salt of the formula IV

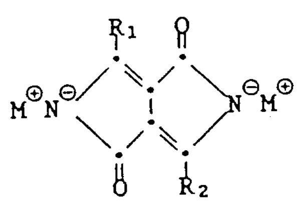

(IV)

in which M is an alkali metal, and subsequently liberating a compound of the formula I by protolysis of a compound of the above formula IV, which comprises adding in stages to 1 part by weight of a compound of the above formula IV in at least two portions, as a protolysing agent, 5-20 parts by weight of water, an inorganic and/or organic acid, a mixture of water and an organic solvent, or a mixture of an inorganic or organic acid and water and/or at least one organic solvent, and reacting the mixture at a temperature from 20 to 150 ° C for 1 to 12 hours after the addition of each of the portions, the portion size varying between 10 and 75 % by weight of the total amount of compound of the formula IV.

**2.** A process according to claim 1, wherein the starting material used is a single-substance nitrile of the formula II or III in which $R_1$ and $R_2$ are phenyl or naphthyl radicals.

**3.** A process according to claim 1, wherein the starting materials used are nitriles of the formula II or III in which $R_1$ and $R_2$, as aromatic N-heterocyclic radicals, are o-, m- or p-pyridyl.

**4.** A process according to claim 1, wherein the starting materials used are nitriles of the formula V

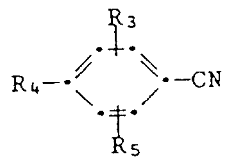

(V)

in which $R_3$, $R_4$ and $R_5$ independently of one another are hydrogen, fluorine, chlorine, bromine, carbamoyl, cyano, trifluoromethyl, $C_1$-$C_8$alkyl, $C_1$-$C_3$alkoxy, $C_1$-$C_3$alkylmercapto, $C_2$-$C_5$alkoxycarbonyl, $C_2$-$C_5$alkanoylamino, N-dimethylamino, N-diethylamino or phenoxy, phenylmercapto or benzoylamino each of which is unsubstituted or substituted by one or two halogen atoms or one or two $C_1$-$C_3$alkyl or $C_1$-$C_3$alkoxy groups, at least one of the substituents $R_3$, $R_4$ and $R_5$ being hydrogen.

**5.** A process according to claim 1, wherein the starting materials used are nitriles of the formula VI

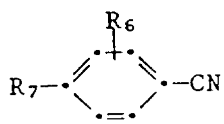

(VI)

in which $R_6$ and $R_7$ independently of one another are hydrogen, chlorine, bromine, fluorine, methyl, methoxy, ethoxy, cyano; or are phenoxy or phenylmercapto each of which is unsubstituted or substituted by chlorine or methyl; or are methoxycarbonyl, ethoxycarbonyl, acetylamino or benzoylamino which is unsubstituted or substituted by chlorine, methyl or methoxy.

**6.** A process according to claim 5, wherein one of the substituents $R_6$ and $R_7$ is as defined in claim 5 and the other is hydrogen.

**7.** A process according to claim 1, wherein the dialkyl succinate used is a symmetrical, branched dialkyl succinate in which each alkyl radical in the succinic acid ester radical has 3 to 5 C atoms.

**8.** A process according to claim 1 wherein the solvent used for the first reaction stage is a secondary or tertiary alcohol.

**9.** A process according to claim 1, wherein the strong base used is an alkali metal alcoholate.

**10.** A process according to claim 1, wherein the reaction in the first reaction stage is carried out at a temperature from 60 to 140 °C.

**11.** A process according to claim 1, wherein the organic solvent used for the protolysis is an alcohol having 1 to 5 C atoms.

**12.** A process according to claim 1, wherein the protolysing agent used is water, mixtures of water and an aliphatic alcohol, water mixed with an acid, or a mixture of water, an acid and an organic solvent.

**13.** A process according to claim 12, wherein water on its own or water/methanol mixtures are used.

**14.** A process according to claim 1, wherein the protolysis stage is carried out in two portions, the first portion of the compound of the formula IV being 40 to 50% by weight of the total amount.

15. A process according to claim 1, wherein the temperature in the protolysis stage is between 50 and 100 ° C.

## Revendications

1. Procède pour préparer des 1,4-dicétopyrrolo-[3,4-c]-pyrroles de formule I

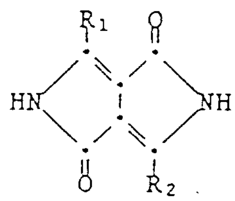

(I),

où $R_1$ et $R_2$, indépendamment l'un de l'autre, représentent des restes phényle, diphényle ou naphtyle ou des restes hétérocycliques aromatiques avec O, S ou N, en faisant réagir 1 mole d'un dicyclohexylester, dialkylester, monoalkylmonophénylester ou diphénylester de l'acide succinique, où dans le reste d'ester de l'acide succinique alkyle signifie un alkyle en $C_1$-$C_{18}$ et phényle signifie un phényle non substitué ou un phényle substitué par un ou deux atomes d'halogène, un ou deux groupes alkyle en $C_1$-$C_6$ ou alcoxy en $C_1$-$c_6$, avec 2 moles d'un nitrile de formule II ou III, ou avec 1 mole d'un nitrile de formule II et 1 mole d'un nitrile de formule III

$R_1$-CN (II) , $R_2$-CN (III),

Où $R_1$ et $R_2$ ont la signification donnée plus haut, dans un solvant organique inerte en présence d'un métal alcalin ou d'un alcoolate alcalin comme base forte, à température élevée, pour obtenir un sel de métal alcalin de formule IV

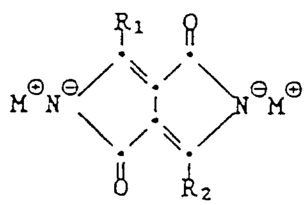

(IV),

où M représente un métal alcalin, puis en libérant un composé de formule I par protolyse d'un composé de la formule précédente IV, caractérisé en ce que l'on additionne de façon séquentielle 1 partie en poids d'un composé de formule précédente IV, en au moins deux portions, de 5-20 parties en poids d'eau, d'un acide inorganique et/ou organique, d'un mélange d'eau et d'un solvant organique ou d'un mélange d'un acide inorganique ou organique et d'eau et/ou d'au moins un solvant organique, comme agent de protolyse, et en ce que, après l'addition des portions respectives, on laisse réagir de 1 à 12 heures à une température de 20 à 150 ° C, la grosseur des portions variant entre 10 et 75% en poids de la quantité totale du composé de formule IV.

2. Procédé selon la revendication 1, caractérisé en ce que l'on utilise comme matière de départ un nitrile unitaire de formule II ou III, où $R_1$ et $R_2$ représentent les radicaux phényle ou naphtyle.

3. Procédé selon la revendication 1, caractérisé en ce que l'on utilise comme matière de départ des nitriles de formule II ou III, où $R_1$ et $R_2$ représentent, comme restes hétérocycliques aromatiques avec M, le o-, m- ou p-pyridyle.

4. Procédé selon la revendication 1, caractérisé en ce que l'on utilise comme matière de départ des nitriles de formule V

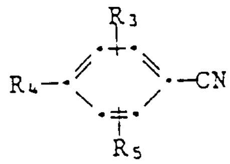 (V)

où $R_3$, $R_4$ et $R_5$, indépendamment les uns des autres, représentent l'hydrogène, le fluor, le chlore, le brome, le carbamoyle, le cyano, le trifluorométhyle, un alkyle en $C_1$-$C_8$, un alcoxy en $C_1$-$C_3$, un alkylmercapto en $C_1$-$C_3$, un alcoxycarbonyle en $C_2$-$C_5$, un alcanoylamino en $C_2$-$C_5$, le N-diméthyl ou diéthylamino, un phénoxy, un phénylmercapto ou un benzoylamino non substitués ou substitués par un ou deux atomes d'halogène, un ou deux groupes alkyle en $C_1$-$C_3$ ou alcoxy en $C_1$-$C_3$, où au moins un des substituants $R_3$, $R_4$ et $R_5$ représente l'hydrogène.

**5.** Procédé selon la revendication 1, caractérisé en ce que l'on utilise comme matière de départ des nitriles de formule VI

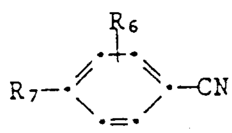 (VI)

où $R_6$ et $R_7$, indépendamment l'un de l'autre, représentent l'hydrogène, le chlore, le brome, le fluor, le méthyle, le méthoxy, l'éthoxy, le cyano, un phénoxy ou un phénylmercapto non substitué ou substitué par le chlore ou le méthyle, le méthoxy ou éthoxycarbonyle, l'acétylamino ou le benzoylamino non substitué ou substitué par le chlore, le méthyle, ou le méthoxy.

**6.** Procédé selon la revendication 5, caractérisé en ce que l'un des substituants $R_6$ et $R_7$ a la signification donnée dans la revendication 5 et l'autre est l'hydrogène.

**7.** Procédé selon la revendication 1, caractérisé en ce que l'on utilise comme dialkylester de l'acide succinique un dialkylester symétrique ramifié de l'acide succinique, où chaque reste alkyle dans le reste d'ester de l'acide succinique a de 3 à 5 atomes de C.

**8.** Procédé selon la revendication 1, caractérisé en ce que l'on utilise comme solvant pour la première étape de réaction un alcool secondaire ou tertiaire.

**9.** Procédé selon la revendication 1, caractérisé en ce que l'on utilise comme base forte un alcoolate alcalin.

**10.** procédé selon la revendication 1, caractérisé en ce que la réaction, lors de la première étape de réaction, est mise en oeuvre à une température de 60 à 140 °C.

**11.** Procédé selon la revendication 1, caractérisé en ce que l'on utilise comme solvant organique pour la protolyse des alcools ayant de 1 à 5 atomes de C.

**12.** Procédé selon la revendication 1, caractérisé en ce que l'on utilise comme agent de protolyse l'eau, des mélanges d'eau et d'un alcool aliphatique, de l'eau en mélange avec un acide, ou un mélange d'eau, d'un acide et d'un solvant organique.

**13.** Procédé selon la revendication 12, caractérisé en ce que l'on utilise de l'eau seule ou des mélanges d'eau et de méthanol.

**14.** Procédé selon la revendication 1, caractérisé en ce que l'étape de protolyse est réalisée en deux portions, où la première portion du composé de formule IV représente de 40 à 50 % en poids de la quantité totale.

15. Procédé selon la revendication 1, caractérisé en ce que la température, Lors de l'étape de protolyse, est entre 50 et 100 ° C.